(19) Europäisches Patentamt
European Patent Office
Office européen des brevets

(11) Veröffentlichungsnummer: **0 028 716**
**B1**

(12) **EUROPÄISCHE PATENTSCHRIFT**

(45) Veröffentlichungstag der Patentschrift: **12.11.86**

(21) Anmeldenummer: **80106211.8**

(22) Anmeldetag: **11.10.80**

(51) Int. Cl.$^4$: **A 61 K 37/02,** C 07 K 5/10, C 07 K 7/06

(54) **Motilitätssteigernde Peptide und deren Verwendung.**

(30) Priorität: **16.10.79 DE 2941790**

(43) Veröffentlichungstag der Anmeldung: **20.05.81 Patentblatt 81/20**

(45) Bekanntmachung des Hinweises auf die Patenterteilung: **12.11.86 Patentblatt 86/46**

(84) Benannte Vertragsstaaten: **AT BE CH DE FR GB IT LI NL SE**

(56) Entgegenhaltungen:
**DE-A-2 739 440**
**FR-A-2 359 120**

**CHEMICAL ABSTRACTS, Band 91, Nr. 5, 30. July 1979 Zusammenfassung 39834d, COLUMBUS, OHIO (US) W. MAYR u.a.: "Synthesis and conformation of a polyoxyethylene-bound undecapeptide of the alamithicin helix and (2-methyl-alanyl-L-alanine)1-7"**

(73) Patentinhaber: **HOECHST AKTIENGESELLSCHAFT**
**Postfach 80 03 20**
**D-6230 Frankfurt am Main 80 (DE)**

(72) Erfinder: **Bickel, Martin, Dr.**
**Beethovenstrasse 40**
**D-6000 Frankfurt am Main (DE)**
Erfinder: **Geiger, Rolf, Prof. Dr.**
**Heinrich-Bleicher-Strasse 33**
**D-6000 Frankfurt am Main 50 (DE)**
Erfinder: **Teetz, Volker, Dr.**
**An der Tann 20**
**D-6238 Hofheim am Taunus (DE)**
Erfinder: **Obermeier, Rainer, Dr.**
**Langenhainer Weg 14**
**D-6234 Hattersheim am Main (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 028 716 B1

**Beschreibung**

Aus der Literatur, wie beispielsweise aus der DE—A1—27 39 440, der FR—A1—2 359 120, der EP—A1—559, der EP—A3—1684 oder der EP—A1—2236 sind Enkephalin-Analoge und deren Verwendung u.a. als Analgetika, Antipsychotika oder Antidiarrhoika bekannt.

Es wurde gefunden, dass Peptide der Formel I

$$A—Tyr—B—C—D—E \quad (I)$$

die Motilität der Muskulatur des Magen-Darmtrakts und der Harnwege stimulieren und darum zur Behandlung von Lähmungserscheinungen dieser Organe verwendet werden können.

In der Formel bedeuten

A Wasserstoff,
Alkyl, Alkenyl oder Alkencycloalkyl mit je bis zu 7 C-Atomen,
eine α-Aminosäure oder ein Peptid mit 2—3 Aminosäuren, vorzugsweise in L-Konfiguration,
B eine D-Aminosäure oder α-Amino-isobuttersäure,
C Glycin oder Azaglycin,
D Phenylalanin, das im Benzolring durch Methyl, Methoxy, Halogen, Nitro- oder Trifluormethyl substituiert sein kenn,
Dehydro- oder Hexahydrophenalanin,
Aza- oder N-Methylphenylalanin,

E (a) $—O—R_1$· oder 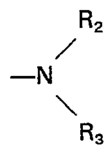

worin $R_1$, $R_2$ und $R_3$ gleich oder verschieden sein können und bedeuten
Wasserstoff,
Alkyl mit 1—6 C-Atomen,
Cycloalkyl mit 3—6 C-Atomen,
Alkencycloalkyl mit 4—8 C-Atomen,
Hydroxyalkyl oder Dihydroxyalkyl, Thioalkyl oder Mercaptoalkyl mit 1—6 C-Atomen,
Aryl mit 6—10 C-Atomen,
Aralkyl mit 7—11 C-Atomen,
oder $R_2$ und $R_3$ zusammen Polymethylen mit 2—6 C-Atomen oder $R_1$ Methyl-polyäthylenglycol mit einem Polymerisationsgrad von bis zu etwa 50,
(b) einen Aminoalkohol oder eine Aminosäure, die ihrerseits eine der unter (a) definierten Gruppen tragen kann,
(c) Homoserin- oder Homocystein-thiolacton.

Die Erfindung betrifft die Verwendung der Peptide der Formel I zur Herstellung eines Arzneimittels zur Steigerung der Motilität der Muskulatur des Magen-Darmtrakts und der Harnwege.

Erhebliche Flüssigkeits- und Gasansammlungen als Folge der fehlenden Spontanmotilitat des Ileus sind für die postoperative Darmatonie charakteristisch. Sie können durch Überdehnung der Darmwand das Einsetzen der Spontanmotorik verhindern. Im weiteren Verlauf kann es durch Elektrolyt- und Flüssigkeitsexsudation in das Darmlumen zu lebensbedrohlichem Kreislaufversagen kommen. Diese Entwicklung wird durch die erfindungsgemässen Peptide verhindert.

Die erfindungsgemässen Peptide stimulieren die Motorik des Magen-Darmtrakts und der ableitenden Harnwege beim Hund und beim Menschen. Sie steigern den Ruhetonus und die segmentale Kontraktionsaktivität des Darmes und fördern dadurch sekundär auch die Wasserresorption aus dem Darmlumen (Ileum).

Sie wirken auch motilitätssteigernd auf die Muskulatur der ableitenden Harnwege ein und sind damit geeignet, Atonien des Magen-Darmkanals wie auch der ableitenden Harnwege zu beseitigen.

Diese Wirkung ist umso überraschender, als die erfindungsgemässen Peptide im Prinzip bereits literaturbekannt sind und als Enkephalin-Analoga am Meerschweinchen-Ileum, dessen Kontraktion sie verhindern, auf diese morphiumähnliche Wirkung geprüft wurden. Es wurde jedoch nicht erkannt, dass sich diese Wirkung speziesabhängig umkehren kann, wodurch diese Verbindungen zu wertvollen Arzneimitteln werden.

Man hat bisher versucht, postoperative Darmatonien, die durch das Fehlen der Spontanmotilität des Darmes gekennzeichnet sind, durch Gabe von Parasympathomimetica zu überwinden. Dabei besteht jedoch immer die Gefahr, dass es zu unerwünschten systemischen cholinergen Effekten kommt, was bei den erfindungsgemäss vorgeschlagenen Peptiden nicht der Fall ist.

Gegenstand der Erfindung ist demgemäss die Verwendung von Peptiden der Formel I zur Steigerung

der Motilität der Muskulatur des Magen-Darmtrakts und der Harnwege sowie zur Behandlung von Lähmungserscheinungen an dieser Muskulatur.

Bei den für die Komponente A der Formel I in Frage kommenden Aminosäuren handelt es sich vorzugsweise um die in Proteinen vorkommenden Aminosäuren sowie deren einfache Metabolite wie Ornithin, Methionin-sulfoxid oder -sulfon, Homocystein oder Pyroglutaminsäure.

Die unter B genannten α-D-Aminosäuren sind vorzugsweise Enantiomere der natürlich vorkommenden Aminosäuren. Sie können in der Seitenkette substituiert sein. Aminogruppen können z.B. durch Alkanoyl, Cycloalkanoyl, Aroyl oder Aralkanoyl mit bis zu 10 C-Atomen substituiert sein, ferner durch Alkyl-, Cycloalkyl- oxycarbonyl mit bis zu 7 C-Atomen oder Benzyl-oxycarbonyl. Hydroxy- oder Mercaptogruppen können durch Alkyl, Aryl oder Aralkyl mit bis zu 10 C-Atomen verethert sein, Carboxylgruppen können die unter E (a) genannten Gruppierungen tragen. Thioether können in Sulfoxid oder Sulfon überführt sein. Aromaten können durch Halogen, Methyl, Methoxy, Nitro- oder Trifluormethyl substituiert sein. Ferner kann die α-D-Aminosäure auch Norleucin sein.

Die unter E genannten α-Aminoalkohole und α-Aminosäuren können sowohl in der L- wie auch in der D-Konfiguration vorliegen. Hinsichtlich der Seitenketten können sie entsprechend den oben unter B genannten Aminosäuren substituiert sein. Als Aminoalkohole kommen in erster Linie die den entsprechenden natürlichen Aminosäuren zugehörigen Reduktionsprodukte in Frage, wie z.B. Prolinol oder Methioninol, das seinerseits auch als Sulfoxid oder Sulfon vorliegen kann.

Als besonders günstig haben sich Verbindungen erwiesen, worin Glied E ein Methyl-polyäthylenglykol mit einem Polymerisationsgrad von bis zu 50 oder einen Aminoalkohol oder eine Aminosäure, der (die) seinerseits (ihrerseits) den genannten Methyl-polyäthylenglykol-Rest trägt, bedeutet. Diese neuen Verbindungen bilden ebenfalls einen Gegenstand der Erfindung.

Sie besitzen die interessante Eigenschaft, die Wirkung der zugrundeliegenden Peptid-Verbindung zu erhöhen und die Löslichkeit zu verbessern, sodass sie es ermöglichen, an sich schwerlösliche Peptide dem erfindungsgemässen Zweck zuzuführen.

Zahlreiche Vertreter der erfindungsgemässen Peptide sind literaturbekannt. Ihre Herstellung folgt den allgemeinen Methoden der Peptidchemie und ist in zahlreichen Veröffentlichungen ausführlich beschrieben.

Die erfindungsgemässen Peptide wirken intravenös bereits in einer Dosierung von 10 ng/kg und können unbedenklich auch in höherer Dosierung angewandt werden. Auch Morphin übt unter geeigneten Bedingungen eine ähnliche Wirkung aus, jedoch erst in einer etwa 50 fach höheren Dosierung. Das parasympathomimetisch wirkende Carbaminoylcholin benötigt für die gleiche Wirkung etwa eine 10 fache Dosierung. So zählen die erfindungsgemässen Peptide zu den stärksten Effektoren der Motorik des Magen-Darmtrakts und der ableitenden Harnwege. Ihre morphinähnliche Wirkung ist zwar bekannt, doch überschreiten bei der geringen erforderlichen Dosierung weder nach intravenöser, subcutaner noch intranasaler Anwendung die wirksamen Dosen die Blut-Hirn-Schranke. Sie üben deshalb in der für den erfindungsgemässen Zweck erforderlichen Menge keine Wirkungen im Zentralnervensystem aus.

Variationen in den Positionen A—D der Peptide ändern die beschriebene Wirkung qualitativ nicht, sondern bewirken nur eine in quantitativer Hinsicht unterschiedliche Erhöhung der Wirkung gegenüber der sehr schwach wirksamen Stammverbindung, dem natürlichen Enkephalin.

Beim Menschen liegt der Dosisbereich in Abhängigkeit von der angewandten Verbindung bei 0,5 bis 500 mcg bei intravenöser und subcutaner Applikation und bei etwa 20 mcg bis 25 mg bei intranasaler Applikation. Die Dosis kann, z.B. bei Resorptionsstörungen, unbedenklich erhöht werden.

Die erfindungsgemässen Verbindungen eignen sich zur Beseitigung postoperativer Atonien des Magen-Darmkanals und der ableitenden Harnwege. Sie können auch bei der Hirschsprung'schen Krankheit, die auf einem angeborenen Megacolon beruht, eingesetzt werden. Ein Vorzug der Therapie mit diesen Verbindungen ist die Möglichkeit, ihre Wirkung durch Opiatantagonisten jederzeit aufheben zu können.

Die nachfolgenden Beispiele zeigen eine charakteristische Synthese sowie die Herstellung von pharmazeutischen Präparaten von Peptiden für die erfindungsgemässe Verwendung.

Beispiel 1

H-Tyr-D-Lys(For)-Gly-Phe-Met-O-$PEG_{3000}$-OMe

a) Boc-Met-O-$PEG_{3000}$-OMe

9,0 g käuflicher Polyethylenglycol-monomethylether mit einem mittleren Molekülargewicht von 3000 ($PEG_{3000}$-OMe) und 5,0 g Boc-Met-OH werden in 50 ml Dimethylformamid unter leichtem Erwärmen gelöst. Nach Zugabe von 2,0 g 1-Hydroxybenzotriazol und 4,5 g Dicyclohexyl-carbodiimid lässt man 42 Stunden bei Raumtemperatur stehen. Dann fällt man mit Ether und wiederholt die Fällung aus Methylenchlord/Ether. Ausbeute 3,5 g.

b) H-Met-O-$PEG_{3000}$-OMe, HCl

Man löst 3,5 g der nach (a) erhaltenen Boc-Verbindung in 10 ml Ameisensäure und gibt 1 ml 6N HCl zu. Nach 30 min. fällt man mit Ether und fällt aus Methylenchlorid/Ether um. Ausbeute 3,2 g.

c) Boc-Tyr(Bu[t])-D-Lys(For)-Gly-Phe-Met-O-$PEG_{3000}$-OMe

3,15 g H-Met-O-$PEG_{3000}$-OMe, HCl und 2,0 g Boc-Tyr(Bu[t])-D-Lys(For)-Gly-Phe-OH, hergestellt nach der Deutschen Patentanmeldung 29 33 947 (HOE 79/F 225), in 15 ml Dimethylformamid werden mit 0,6 ml N-Ethylmorpholin, 0,5 g 1-Hydroxybenzotriazol und 0,75 g Dicyclohexyl-carbodiimid unter Rühren versetzt. Nach 48 h Rühren bei Raumtemperatur fällt man mit Ether und wiederholt die Fällung aus Methylenchlorid/Ether. Ausbeute 2,7 g.

d) H-Tyr-D-Lys(For)-Gly-Phe-Met-O-$PEG_{3000}$-OMe-hydrochlorid

Man löst 2,5 g der nach (c) erhaltenen Verbindung in 12 ml Ameinsensäure und versetzt unter Rühren bei 0°C mit 1,2 ml 6N HCl. Nach 40 min. fällt man mit Ether und weiderholt die Fällung aus Methylenchlorid/Ether, verreibt den kristallinen Niederschlag mit Ether und trocknet im Vakuum über Schwefelsäure. Ausbeute 2,2 g. Die Aminosäurenanalyse entspricht in Zusammensetzung und Peptidgehalt dem erwarteten Produkt.

Beispiel 2

1 mg Tyr-D-Met-Gly-Phe-methioninol-sulfoxid oder Tyr-D-Lys-(For)-Gly-Phe-homocystein-thiolacton wird in 1 l physiologischer Kochsalzlösung aufgelöst, die 1% Phenylethanol enthält. Man filtriert steril und füllt in Ampullen zu 1 ml ab.

Beispiel 3

Man löst 250 mg $CH_3$-Tyr-D-Met-Gly-Phe-Met-$NH_2$ oder Tyr-D-Ser-Gly-Phe-Pro-OH in 1 l physiologischer Kochsalzlösung, die 1% Phenylethanol enthält. Die Lösung wird steril filtriert und in Sprühfläschchen abgefüllt, die pro Sprühstoss 0,2 ml Lösung abgeben.

**Patentansprüche für die Vertragsstaaten: BE CH DE FR GB IT LI NL SE**

1. Peptide der Formel I

$$A—Tyr—B—C—D—E \qquad (I)$$

in welcher bedeuten:

A Wasserstoff, Alkyl, Alkenyl oder Alkencycloalkyl mit je bis zu 7 C-Atomen, eine α-Aminosäure oder ein Peptide mit 2—3 Aminosäuren, vorzugsweise in L-Konfiguration,
B eine D-Aminosäure oder α-Amino-isobuttersäure,
C Glycin oder Azaglycin,
D Phenylalanin, das im Benzolring durch Methyl, Methoxy, Halogen, Nitro oder Trifluormethyl substituiert sein kann, Dehydro- oder Hexahydrophenylalanin Aza- oder N-Methylphenylalanin,
E a) O—$R_1$, worin $R_1$ für einen Methyl-polyethylenglycol-Rest mit einem Polymerisationsgrad bis zu etwa 50 steht, oder
b) einen Aminoalkohol oder eine Aminosäure, die ihrerseits die unter (a) definierte Gruppe trägt.

2. Peptid der Formel I gemäß Anspruch I, in welcher bedeuten:

A Wasserstoff, Alkyl, Alkenyl oder Alken cycloalkyl mit je bis zu 7 C-Atomen, eine in Proleinen vorkommende α-L-Aminosäure bzw. deren einfacher Metabolit oder ein aus diesen Aminosäuren aufgebautes Di- oder Tripeptid,
B eine zu natürlich vorkommende Aminosäuren enantiomere α-D-Aminosäure,
die in der Seitenkette
an einer gegebenenfalls vorhandenen Aminogruppe durch Alkanoyl, Cycloalkanoyl, Aroyl oder Aralkanoyl mit bis zu 10 C-Atomen, ferner durch Alkyl-, Cycloalkyl-oxycarbonyl mit bis zu 7 C-Atomen oder Benzyl-oxycarbonyl substituiert sein kann,
an gegebenenfalls vorhandenen Hydroxy- oder Mercaptogruppen durch Alkyl, Aryl oder Aralkyl mit jeweils bis zu 10 C-Atomen verethert sein kann und
an Carboxylgruppen die Gruppierungen

$$—O—R_1 \quad \text{oder} \quad —N\begin{matrix}R_2\\R_3\end{matrix}$$

tragen kann, worin $R_1$, $R_2$ und $R_3$ gleich oder verschieden sein können und
Wasserstoff, Alkyl mit 1—6 C-Atomen Cycloalkyl mit 3—6 C-Atomen, Alkencycloalkyl mit 4—8

C-Atomen, Hydroxyalkyl oder Dihydroxyalkyl, Thioalkyl oder Mercaptoalkyl mit 1—6 C-Atomen, Aryl mit 6—10 C-Atomen, Aralkyl mit 7—11 C-Atomen,

bedeuten und $R_2$ und $R_3$ zusätzlich zusammen Polymethylen mit 2—6 C-Atomen bedeuten können und $R_1$ zusätzlich Methyl-polyethylenglykol mit einem Polymerisationsgrad von bis zu etwa 50 bedeuten kann, und

deren gegebenenfalls vorhandene Thioetherfunktionen in Sulfoxid oder Sulfon überführt und deren gegebenenfalls vorhandene aromatischen Reste durch Halogen, Methyl, Methoxy, Nitro oder Trifluormethyl substituiert sein können,

Norleucin oder α-Amino-isobuttersäure,

C Glycin oder Azaglycin,

D Phenylalanin, das im Benzolring durch Methyl, Methoxy, Halogen, Nitro oder Trifluormethyl substituiert sein kann, Dehydro- oder Hexahydrophenalanin Aza- oder N-Methylphenylalanin bedeuten und

E a) wie im Anspruch 1 unter (a) definiert ist, oder

b) für einen α-Aminoalkohol oder eine α-Aminosäure jeweils in der L- oder D-Konfiguration steht, der (die) gegebenenfalls in der Seitenkette wie die Aminosäure B substituiert ist und die ihrerseits die unter (a) definierte Gruppe trägt.

3. H-Tyr-D-Lys(For)-Gly-Phe-Met-O-$PEG_{3000}$-OMe oder dessen Hydrochlorid.

4. Peptid gemäß einem der Ansprüche 1 bis 3 zur Anwendung als Heilmittel.

5. Peptid gemäß einem der Ansprüche 1 bis 3 zur Anwendung als Mittel zur Steigerung der Motilität der Muskulatur des Magen-Darmtrakte und der Harnwege.

6. Peptid gemäß einem der Ansprüche 1 bis 3 zur Verwendung als Mittel zur Behandlung von Lähmungserscheinungen des Magen-Darmtraktes und der Harnwege.

7. Verfahren zur Herstellung eines Peptides gemäß einem der Ansprüche 1 bis 6, dadurch gekennzeichnet, daß man Aminosäuresequenzen der Formel I nach Methoden der Peptidsynthese aufbaut.

8. Verwendung eines Peptids der Formel I

A—Tyr—B—C—D—E (I)

in welcher bedeuten

A Wasserstoff, Alkyl, Alkenyl oder Alkencycloalkyl mit je bis zu 7 C-Atomen, eine α-Aminosäure oder ein Peptid mit 2—3 Aminosäuren, vorzugsweise in L-Konfiguration,

B eine D-Aminosäure oder α-Amino-isobuttersäure,

C Glycin oder Azaglycin,

D Phenylalanin, des im Benzolring durch Methyl, Methoxy, Halogen, Nitro oder Trifluormethyl substituiert sein kann, Dehydro- oder Hexahydrophenalanin Aza- oder N-Methylphenylalanin,

E (a) —O—$R_1$ oder 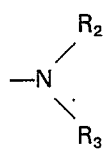

worin $R_1$, $R_2$ und $R_3$ gleich oder verschieden sein können und bedeuten Wasserstoff, Alkyl mit 1—6 C-Atomen, Cycloalkyl mit 3—6 C-Atomen, Alkencycloalkyl mit 4—8 C-Atomen, Hydroxyalkyl oder Dihydroxyalkyl, Thioalkyl oder Mercaptoalkyl mit 1—6 C-Atomen, Aryl mit 6—10 C-Atomen, Aralkyl mit 7—11 C-Atomen, oder $R_2$ und $R_3$ zusammen Polymethylen mit 2—6 C-Atomen, oder $R_1$ Methyl-polyäthylenglycol mit einem Polymerisationsgrad von bis zu etwa 50,

(b) einen Aminoalkohol oder eine Aminosäure, die ihrerseits eine der unter (a) definierten Gruppen tragon kann,

(c) Homoserin- oder Homocystein-Thiolacton,

zur Herstellung eines Arzneimittels zur Steigerung der Motilität der Muskulatur des Magen-Darmtrakts und der Harnwege.

9. Verwendung eines Peptids der Formel I, worin A, B, C und D wie im Anspruch 2 definiert sind und E wie im Anspruch 1 unter (a) oder (c) definiert ist oder für einen α-Aminoalkohol oder eine α-Aminosäure jeweils in der L- oder D-Konfiguration steht, der (die) gegebenenfalls in der Seitenkette wie die Aminosäure B substituiert ist und der (die) ihrerseits eine der unter (a) definierten Gruppen tragen kann, zur Herstellung eines Arzneimittels zur Steigerung der Motilität der Muskulatur des Magen-Darmtrakts und der Harnwege.

10. Verwendung eines Peptids der Formel I, worin A, B, C, D und E wie im Anspruch 8 oder 9 definiert sind, zur Herstellung eines Arzneimittels zur Behandlung von Lähmungserscheinungen des Magen-Darmtrakts und der Harnwege.

**Patentansprüche für den Vertragsstaat: AT**

1. Verfahren zur Herstellung eines Peptids der Formel I

A—Tyr—B—C—D—E (I)

in welcher bedeuten:

A Wasserstoff, Alkyl, Alkenyl oder Alkencycloalkyl mit je bis zu 7 C-Atomen, eine α-Aminosäure oder ein Peptid mit 2—3 Aminosäuren, vorzugsweise in L-Konfiguration,
B eine D-Aminosäure oder α-Amino-isobuttersäure,
C Glycin oder Azaglycin,
D Phenylalanin, das im Benzolring durch Methyl, Methoxy, Halogen, Nitro oder Trifluormethyl substituiert sein kann, Dehydro- oder Hexahydrophenalanin Aza- oder N-Methylphenylalanin,
E a) O—$R_1$, worin $R_1$ für einen Methyl-polyethylenglycol-Rest mit einem Polymerisationsgrad bis zu etwa 50 steht, oder
b) einen Aminoalkohol oder eine Aminosäure, die ihrerseits die unter (a) definierte Gruppe trägt,

dadurch gekennzeichnet, daß man Aminosäuresequenzen der genannten Formel nach Methoden der Peptidsynthese aufbaut.

2. Verfahren gemäß Anspruch 1, dadurch gekennzeichnet, daß ein Peptid der Formel I aufgebaut wird, worin

A Wasserstoff, Alkyl, Alkenyl oder Alkencycloalkyl mit je bis zu 7 C-Atomen, eine in Proleinen vorkommende α-L-Aminosäure bzw. deren einfacher Metabolit oder ein aus diesen Aminosäuren aufgebautes Di- oder Tripeptid,
B eine zu natürlich vorkommende Aminosäuren enantiomere α-D-Aminosäure,
die in der Seitenkette
an einer gegebenenfalls vorhandenen Aminogruppe durch Alkanoyl, Cycloalkanoyl, Aroyl oder Aralkanoyl mit bis zu 10 C-Atomen, ferner durch Alkyl-, Cycloalkyl-oxycarbonyl mit bis zu 7 C-Atomen oder Benzyl-oxycarbonyl substituiert sein kann,
an gegebenenfalls vorhandenen Hydroxy- oder Mercaptogruppen durch Alkyl, Aryl oder Aralkyl mit jeweils bis zu 10 C-Atomen verethert sein kann und
an Carboxylgruppen die Gruppierungen

—O—$R_1$ oder 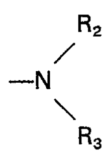

tragen kann, worin $R_1$, $R_2$ und $R_3$ gleich oder verschieden sein können und
Wasserstoff, Alkyl mit 1—6 C-Atomen Cycloalkyl mit 3—6 C-Atomen, Alkencycloalkyl mit 4—8 C-Atomen, Hydroxyalkyl oder Dihydroxyalkyl, Thioalkyl oder Mercaptoalkyl mit 1—6 C-Atomen, Aryl mit 6—10 C-Atomen, Aralkyl mit 7—11 C-Atomen, bedeuten und
$R_2$ und $R_3$ zusammen zusätzlich Polymethylen mit 2—6 C-Atomen bedeulen können und zusätzlich Methyl-polyethylenglykol mit einem Polymerisationsgrad von bis zu etwa 50 bedeuten kann, und
deren gegebenenfalls vorhandene Thioetherfunktionen in Sulfoxid oder Sulfon überführt und deren gegebenenfalls vorhandene aromatischen Reste durch Halogen, Methyl, Methoxy, Nitro oder Trifluormethyl substituiert sein können, Norleucin oder α-Amino-isobuttersäure,
C Glycin oder Azaglycin,
D Phenylalanin, das im Benzolring durch Methyl, Methoxy, Halogen, Nitro oder Trifluormethyl substituiert sein kann, Dehydro oder Hexahydrophenylalanin Aza- oder N-Methylphenylalanin bedeuten und
E a) wie im Anspruch 1 unter (a) definiert ist, oder
b) für einen α-Aminoalkohol oder eine α-Aminosäure jeweils in der L- oder D-Konfiguration steht, der (die) gegebenenfalls in der Seitenkette wie die Aminosäure B substituiert ist und der (die) ihrerseits die unter (a) definierte Gruppe trägt.

3. Verfahren gemäß einem der Ansprüche 1 oder 2, dadurch gekennzeichnet, daß

H-Tyr-D-Lys(For)-Gly-Phe-Met-O-$PEG_{3000}$-OMe

hergestellt wird.

4. Verfahren gemäß einem der Ansprüche 1 bis 3 zur Herstellung eines Peptids der Formel I zur Verwendung als Heilmittel.

5. Verfahren gemäß einem der Ansprüche 1 bid 3 zur Herstellung eines Peptids der Formel I zur Verwendung als Mittel zur Steigerung der Motilität der Muskulatur des Magen-Darmtrakts und der Harnwege.

6. Verfahren gemäß einem der Ansprüche 1 bis 3 zur Herstellung eines Peptids der Formel I zur

Verwendung als Mittel zur Behandlung von Lähmungserscheinungen des Magen-Darmtraktes und der Harnwege.

7. Verwendung eines Peptids der Formel I

$$A—Tyr—B—C—D—E \quad (I)$$

in welcher bedeuten

A Wasserstoff, Alkyl, Alkenyl oder Alkencycloalkyl mit je bis zu 7 C-Atomen, eine α-Aminosäure oder eine Peptid mit 2—3 Aminosäuren, vorzugsweise in L-Konfiguration,
B eine D-Aminosäure oder α-Amino-isobuttersäure,
C Glycin oder Azaglycin,
D Phenylalanin, das im Benzolring durch Methyl, Methoxy, Halogen, Nitro oder Trifluormethyl substituiert sein kann, Dehydro- oder Hexahydrophenalanin Aza- oder N-Methylphenylalanin,

E (a) $—O—R_1$ oder 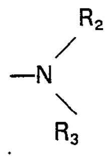

worin $R_1$, $R_2$ und $R_3$ gleich oder verschieden sein können und bedeuten

Wasserstoff, Alkyl mit 1—6 C-Atomen, Cycloalkyl mit 3—6 C-Atomen, Alkencycloalkyl mit 4—8 C-Atomen, Hydroxyalkyl oder Dihydroxyalkyl, Thioalkyl oder Mercaptoalkyl mit 1—6 C-Atomen, Aryl mit 6—10 C-Atomen, Aralkyl mit 7—11 C-Atomen,

oder $R_2$ und $R_3$ zusammen Polymethylen mit 2—6 C-Atomen, oder $R_1$ Methyl-polyäthylenglycol mit einem Polymerisationsgrad von bis zu etwa 50,

(b) einen Aminoalkohol oder eine Aminosäure, die ihrerseits eine der unter (a) definierten Gruppen tragon kann,

(c) Homoserin- oder Homocystein-Thiolacton, zur Herstellung eines Arzneimittels zur Steigerung der Motilität der Muskulatur des Magen-Darmtrakts und der Harnwege.

8. Verwendung eines Peptids der Formel I, worin A, B, C und D wie im Anspruch 2 definiert sind und E wie im Anspruch 1 unter (a) oder (c) definiert ist oder für einen α-Aminoalkohol oder eine α-Aminosäure jeweils in der L- oder D-Konfiguration steht, der (die) gegebenenfalls in der Seitenkette wie die Aminosäure B substituiert ist und der (die) ihrerseits eine der unter (a) definierten Gruppen tragen kann, zur Herstellung eines Arzneimittels zur Steigerung der Motilität der Muskulatur des Magen-Darmtrakts und der Harnwege.

9. Verwendung eines Peptids der Formel I, worin A, B, C, D und E wie im Anspruch 7 oder 8 definiert sind, zur Herstellung eines Arzneimittels zur Behandlung von Lähmungserscheinungen des Magen-Darmtrakts und der Harnwege.

**Revendications pour les Etats Contractants: BE, CH, DE, FR, GB, IT, LI, NL, SE**

1. Peptide de formule I

$$A—Tyr—B—C—D—E \quad (I)$$

dans laquelle:

A représente un atome d'hydrogène, un groupe alkyle, alcényle ou alcène-cycloalkyle ayant chacun jusqu'à 7 atomes de carbone, un acide α-aminé ou un peptide à 2—3 acides aminés, de préférence sous la forme L;
B représente un acide D-aminé ou l'acide α-amino-isobutyrique;
C représente la glycine ou l'azaglycine;
D représente la phénylalanine, qui peut être substituée dans le noyau benzénique par un groupe méthyle, méthoxy, un halogène, un groupe nitro ou trifuorométhyle, la déhydro- ou l'hexahydrophénylalanine, l'aza- ou la N-méthylphénylalanine;
E représente
a) $O—R_1$, où $R_1$ est un reste méthyl-polyéthylèneglycol ayant un degré de polymérisation allant jusqu'à 50 environ, ou
b) un amino-alcool ou un acide aminé, qui de son côté porte le groupe défini sous (a).

2. Peptide de formule I selon la revendication 1, dans lequel:

A représente un atome d'hydrogène, un groupe alkyle, alcényle ou alcène-cycloalkyle ayant chacun jusqu'à 7 atomes de carbone, un acide α-L-aminé présent dans des protéines ou un métabolite simple de celui-ci, ou bien un di- ou un tripeptide édifié à partir de ces acides aminés;

B représente un acide α-D-aminé énantiomère d'acides aminés existant dans la nature, qui dans la chaîne latérale peut être substitué sur un groupe amino éventuellement présent, par un radical alcanoyle, cyclo-alcanoyle, aroyle ou aralcanoyle, ayant jusqu'à 10 atomes de carbone, en outre par un radical alkyl- ou cycloalkyl-oxycarbonyle ayant jusqu'à 7 atomes de carbone ou par un radical benzyloxy-carbonyle, peut être éthérifié sur des groupes hydroxy ou mercapto, éventuellement présente, par un radical alkyle, aryle ou aralkyle ayant chacun jusqu'à 10 atomes de carbone, et qui peut porter sur les groupes carboxy les groupements

$-O-R_1$ ou 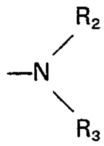

dans lesquels $R_1$, $R_2$ et $R_3$ peuvent être identiques ou différents et représentent chacun un atome d'hydrogène, un groupe alkyle ayant de 1 à 6 atomes de carbone, un groupe cycloalkyle ayant de 3 à 6 atomes de carbone, alcène-cycloalkyle ayant de 4 à 8 atomes de carbone, hydroxyalkyle ou dihydroxyalkyle, thio-alkyle ou mercaptoalkyle ayant de 1 à 6 atomes de carbone, aryle ayant de 6 à 10 atomes de carbone, aralkyle contenant de 7 à 11 atomes de carbone, et $R_2$ et $R_3$ peuvent en outre former ensemble un groupement polyméthylène contenant 2 à 6 atomes de carbone, et $R_1$ peut en outre représenter un méthyl-polyéthylèneglycol ayant un degré de polymérisation allant jusqu'à 50 environ, et dont les fonctions thio-éther éventuellement présentes peuvent être transformées en sulfoxydes ou en sulfones, et dont les restes aromatiques éventuellement présents peuvent être substitués par des atomes d'halogène, des groupes méthyle, méthoxy, nitro ou trifluorométhyle; la norleucine ou l'acide α-amino-isobutyrique;

C est la glycine ou l'azaglycine;

D représente la phénylalanine, qui peut être substituée dans le noyau benzénique par un groupe méthyle, méthoxy, un atome d'halogène, un groupe nitro ou trifluorométhyle; la déhydro- ou l'hexahydrophénylalanine; l'aza- ou la N-méthylphénylalanine; et

E est
- a) tel que défini en (a) dans la revendication 1, ou
- b) représente un α-aminoalcool ou un acide α-aminé, chacun sous la forme L ou D, qui est éventuellement substitué dans la chaîne latérale comme l'acide aminé B et qui, de son côté, porte le groupe défini en (a).

3. H-Tyr-D-Lys(For)-Gly-Phe-Met-O-$PEG_{3000}$-OMe ou son chlorhydrate.

4. Peptide selon l'une des revendications 1 à 3, pour l'utilisation en tant que médicament.

5. Peptide selon l'une des revendications 1 à 3, pour l'utilisation en tant que médicament pour l'augmentation de la motilité des muscles du tractus gastro-intestinal et des voies urinaires.

6. Peptide selon l'une des revendications 1 à 3, pour l'utilisation en tant qu'agent pour le traitement de phénomènes de parésie du tractus gastro-intestinal et des voies urinaires.

7. Procédé pour la préparation d'un peptide selon l'une des revendications 1 à 6, caractérisé par le fait que l'on construit des séquences d'acides aminés de formule I selon des méthodes de la synthèse des peptides.

8. Utilisation d'un peptide de formule I

$$A—Tyr—B—C—D—E \qquad (I)$$

dans lequel

A représente un atome d'hydrogène, un groupe alkyle, alcényle ou alcène-cycloalkyle ayant chacun jusqu'à 7 atomes de carbone, un acide α-aminé ou un peptide à 2—3 acides aminés, de préférence sous la forme L;

B représente un acide D-aminé ou l'acide α-amino-isobutyrique;

C représente la glycine ou l'azaglycine;

D représente la phénylalanine, que peut être substituée dans le noyau benzénique par un groupe méthyle, méthoxy, un halogène, un groupe nitro ou trifluorométhyle; la déhydro- ou l'hexahydrophénylalanine; l'aza- ou la N-méhylphénylalanine;

E représente

(a) $-O-R_1$ ou 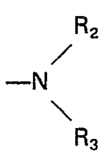

où $R_1$, $R_2$ et $R_3$ peuvent être identiques ou différents et représentent chacun un atome d'hydrogène, un groupe alkyle ayant de 1 à 6 atomes de carbone, un groupe cycloalkyle ayant de 3 à 6 atomes de carbone, un groupe alcène-cycloalkyle ayant de 4 à 8 atomes de carbone, un groupe hydroxyalkyle ou dihydroxyalkyle, thio-alkyle ou mercapto-alkyle ayant de 1 à 6 atomes de carbone, un groupe aryle contenant de 6 à 10 atomes de carbone, un groupe aralkyle ayant de 7 à 11 atomes de carbone, ou $R_2$ et $R_3$ forment ensemble ou groupe polyméthylène ayant de 2 à 6 atomes de carbone, ou $R_1$ est un méthyl-polyéthylèneglycol ayant un degré de polymérisation allant jusqu'à 50 environ;

(b) un aminoalcool ou un acide aminé, qui de son côté peut porter un des groupes définis sous (a),

(c) l'homosérine- ou l'homocystéine-thiolactone, pour la préparation d'un médicament pour l'augmentation de la motilité des muscles du tractus gastro-intestinal et des voies urinaires.

9. Utilisation d'un peptide de formule I, dans lequel A, B, C et D sont tels que définis dans la revendication 2 et E est tel que défini en (a) ou (c) dans la revendication 1 ou représente ou α-aminoalcool ou un acide α-aminé, chacun sous la forme L ou D, qui est éventuellement substitué dans la chaîne latérale comme l'acide aminé B, et qui, de son côté, peut porter un des groupes définis en (a), pour la préparation d'un médicament pour l'augmentation de la motilité des muscles du tractus gastro-intestinal et des voies urinaires.

10. Utilisation d'un peptide de formule I, dans lequel A, B, C, D et E sont tels que définis dans la revendication 8 ou 9, pour la préparation d'un médicament pour le traitement de phénomènes de parésie du tractus gastro-intestinal et des voies urinaires.

**Revendications pour l'Etat Contractant: AT**

1. Procédé pour la préparation d'un peptide de formule I

$$A\text{—}Tyr\text{—}B\text{—}C\text{—}D\text{—}E \qquad (I)$$

dans laquelle:

A représente un atome d'hydrogène, un groupe alkyle, alcényle ou alcène-cycloalkyle ayant chacun jusqu'à 7 atomes de carbone, un acide α-aminé ou un peptide à 2—3 acides aminés, de préférence sous la forme L;

B représente un acide D-aminé ou l'acide α-amino-isobutyrique;

C représente la glycine ou l'azaglycine;

D représente la phénylalanine, qui peut être substituée dans le noyau benzénique par un groupe méthyle, méthoxy, un halogène, un groupe nitro ou trifluorométhyle; la déhydro- ou l'hexahydrophénylalanine; l'aza- ou la N-méthylphénylalanine;

E représente

a) $O\text{—}R_1$ où $R_1$ est un reste méthyl-polyéthylèneglycol ayant un degré de polymérisation allant jusqu'à 50 environ, ou

b) un amino-alcool ou un acide aminé, qui de son côté porte le groupe défini pour (a),

caractérisé par le fait que l'on construit les séquences d'acides aminés de formule indiquée, selon les méthodes de la synthèse des peptides.

2. Procédé selon la revendication 1, caractérisé par le fait que l'on construit un peptide de formule I, dans lequel

A représente un atome d'hydrogène, un groupe alkyle, alcényle ou alcène-cycloalkyle ayant chacun jusqu'à 7 atomes de carbone, un acide α-L-aminé présent dans des protéines ou un métabolite simple de celui-ci, ou bien un di- ou un tripeptide édifié à partir de ces acides aminés;

B représente un acide α-D-aminé énantiomère d'acides aminés existant dans la nature, qui dans la chaîne latérale peut être substitué sur un groupe amino éventuellement présent, par un radical alkanoyle, cyclo-alcanoyle, aroyle ou aralcanoyle, ayant jusqu'à 10 atomes de carbone, en outre par un radical alkyl- ou cycloalkyl-oxycarbonyle ayant jusqu'à 7 atomes de carbone ou par un radical benzyloxy-carbonyle, peut être éthérifié sur des groupes hydroxy, mercapto, éventuellement présente, par un radical alkyle, aryle ou aralkyle ayant chacun jusqu'à 10 atomes de carbone, et qui peut porter sur les groupes carboxy les groupements

$$\text{—O—}R_1 \quad \text{ou} \quad \text{—N}\begin{matrix} / R_2 \\ \backslash R_3 \end{matrix}$$

dans lesquels $R_1$, $R_2$ et $R_3$ peuvent être identiques ou différents et représentent chacun un atome d'hydrogène, un groupe alkyle ayant de 1 à 6 atomes de carbone, un groupe cycloalkyle ayant de 3 à 6

atomes de carbone, alcène-cycloalkyle ayant de 4 à 8 atomes de carbone, hydroxyalkyle ou dihydroxyalkyle, thio-alkyle ou mercaptoalkyle ayant de 1 à 6 atomes de carbone, aryle ayant de 6 à 10 atomes de carbone, aralkyle contenant de 7 à 11 atomes de carbone, et $R_2$ et $R_3$ peuvent en outre former ensemble un groupement polyméthylène contenant de 2 à 6 atomes de carbone, et $R_1$ peut en outre représenter un méthyl-polyéthylèneglycol ayant un degré de polymérisation allant jusqu'à 50 environ et dont les fonctions thio-éther éventuellement présentes peuvent être transformées en sulfoxydes ou en sulfones, et dont les restes aromatiques éventuellement présents peuvent être substitués par des atomes d'halogène, des groupes méthyle, méthoxy, nitro ou trifluorométhyle; la norleucine ou l'acide α-amino-isobutyrique;

C est la glycine ou l'azaglycine;

D représente la phénylalanine, qui peut être substituée dans le noyau benzénique par un groupe méthyle, méthoxy, un atome d'halogène, un groupe nitro ou trifluorométhyle; la déhydro- ou l'hexahydro-phénylalanine; l'aza- ou la N-méthylphénylalanine; et

E est

a) tel que défini en (a) dans la revendication 1, ou

b) représente un α-aminoalcool ou un acide α-aminé, chacun sous la forme L ou D, qui est éventuellement substitué dans la chaîne latérale comme l'acide aminé B et qui, de son côté, porte le groupe défini en (a).

3. Procédé selon l'une des revendications 1 ou 2, caractérisé par le fait que l'on prépare

H-Tyr-D-Lys(For)-Gly-Phe-Met-O-$PEG_{3000}$-OMe.

4. Procédé selon l'une des revendications 1 à 3, pour la préparation d'un peptide de formule I pour l'utilisation en tant que médicament.

5. Procédé selon l'une des revendications 1 à 3, pour la préparation d'un peptide de formule I pour l'utilisation en tant que médicament pour l'augmentation de la motilité des muscles du tractus gastro-intestinal et des voies urinaires.

6. Procédé selon l'une des revendications 1 à 3, pour la préparation d'un peptide de formule I pour l'utilisation en tant que médicament pour le traitement de phénomènes de parésie du tractus gastro-intestinal et des voies urinaires.

7. Utilisation d'un peptide de formule I

A—Tyr—B—C—D—E (I)

dans laquelle:

A représente un atome d'hydrogène, un groupe alkyle, alcényle ou alcène-cycloalkyle ayant chacun jusqu'à 7 atomes de carbone, un acide α-aminé ou un peptide à 2—3 acides aminés, de préférence sous la forme L;

B représente un acide D-aminé ou l'acide α-amino-isobutyrique;

C représente la glycine ou l'azaglycine;

D représente la phénylalanine, qui peut être substituée dans le noyau benzénique par un groupe méthyle, méthoxy, un halogène, un groupe nitro ou trifluorométhyle, la déhydro- ou l'hexahydrophénylalanine, l'aza- ou la N-méthylphénylalanine;

E représente

(a) —O—$R_1$ ou 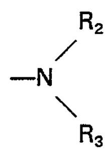

où $R_1$, $R_2$ et $R_3$ peuvent être identiques ou différents et représentent chacun un atome d'hydrogène, un groupe alkyle ayant de 1 à 6 atomes de carbone, un groupe cycloalkyle ayant de 3 à 6 atomes de carbone, un groupe alcène-cycloalkyle ayant de 4 à 8 atomes de carbone, un groupe hydroxyalkyle ou dihydroxyalkyle, thio-alkyle ou mercapto-alkyle ayant de 1 à 6 atomes de carbone, un groupe aryle contenant de 6 à 10 atomes de carbone, un groupe aralkyle ayant de 7 à 11 atomes de carbone, ou $R_2$ et $R_3$ forment ensemble un groupe polyméthylène ayant de 2 à 6 atomes de carbone, ou $R_1$ est un méthyl-polyéthylèneglycol ayant un degré de polymérisation allant jusqu'à 50 environ;

(b) un aminoalcool ou un acide aminé, qui de son côté peut porter un des groupes définis en (a),

(c) l'homosérine- ou l'homocystéine-thiolactone, pour la préparation d'un médicament pour l'augmentation de la motilité des muscles du tractus gastro-intestinal et des voies urinaires.

8. Utilisation d'un peptide de formule I, dans lequel A, B, C et D sont tels que définis dans la revendication 2 et E est tel que défini en (a) ou (c) dans la revendication 1 ou représente un α-aminoalcool

ou un acide α-aminé, chacun sous la forme L ou D, qui est éventuellement substitué dans la chaîne latérale comme l'acide aminé B, et qui, de son côté, peut porter un des groupes définis en (a), pour la préparation d'un médicament pour l'augmentation de la motilité des muscles du tractus gastro-intestinal et des voies urinaires.

9. Utilisation d'un peptide de formule I, dans lequel A, B, C, D et E sont tels que définis dans la revendication 7 ou 8, pour la préparation d'un médicament pour le traitement de phénomènes de parésie du tractus gastro-intestinal et des voies urinaires.

**Claims for the Contracting States: BE, CH, DE, FR, GB, IT, LI, NL, SE**

1. The peptide of the formula I

$$A—Tyr—B—C—D—E \qquad (I)$$

in which

A denotes hydrogen, alkyl, alkenyl, or alkenylcycloalkyl each having up to 7 carbon atoms, an α-amino acid or a peptide with 2 or 3 amino acids, preferably of the L-configuration,
B denotes an α-amino acid or α-amino-isobutyric acid,
C denotes glycine or azaglycine,
D denotes phenylalanine which can be substituted in the benzene nucleus by methyl, methoxy, halogen, nitro or trifluormethyl, dehydro- or hexahydrophenylalanine, aza- or N-methylphenylalanine, and
E denotes
(a) —O—$R_1$ $R_1$ denotes a methyl-polyethylene glycol radical having a degree of polymerization of up to about 50,
(b) an amino alcohol or an amino acid, which on its part may carry one of the groups defined sub (a).

2. The peptide of the formula I according to claim 1 in which

A denotes hydrogen, alkyl, alkenyl or alkenylcycloalkyl each having up to 7 carbon atoms, an α-amino acid occurring in proteins or a simple metabolite thereof, or a di- or tripeptide consisting of these amino acids,
B denotes an α-D-amino acid which is an enantiomer of an amino acid occurring in nature, in the side chain of which an optionally existing amino group can be substituted by alkanoyl, cycloalkanoyl, aroyl or aralkanoyl each having up to 10 carbon atoms, by alkyl-, cycloalkyl- or aralkyl-oxycarbonyl each having up to 7 carbon atoms

optionally existing hydroxy group or mercapto groups can be etherified by alkyl, aryl or aralkyl each having up to 10 carbon atoms, and carboxy groups can carry the groupings

—O—$R_1$ or 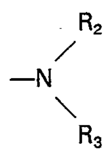

in which $R_1$, $R_2$ and $R_3$ are identical or different and are hydrogen, alkyl with 1 to 6 carbon atoms, cycloalkyl with 3 to 6 carbon atoms, alkenylcycloalkyl with 4 to 8 carbon atoms, hydroxyalkyl or dihydroxyalkyl, thioalkyl or mercaptoalkyl with 1 to 6 carbon atoms, aryl with 6 to 10 carbon atoms, aralkyl with 7 to 11 carbon atoms, and in addition, $R_2$ and $R_3$ can together denote polymethylene with 2 to 6 carbon atoms, and further, $R_1$ can denote methyl-polyethylene glycol having a degree of polymerization of up to about 50, in which optionally existing thioether functions can be converted to sulfoxide or sulfone and optionally existing aromatic radicals can be substituted by halogen, methyl, methoxy, nitro, or trifluoromethyl, norleucine or α-amino-isobutyric acid,
C denotes glycine or azaglycine,
D denotes phenylalanine which can be substituted in the benzene nucleus by methyl, methoxy, halogen, nitro or trifluormethyl, dehydro- or hexahydrophenylalanine, aza- or N-methylphenylalanine, and
E a) is as defined in claim 1 sub (a) or
b) is an α-amino alcohol or an α-amino acid each in the L- or D-configuration, the side chain of which is optionally substituted like amino acid B and which on its part can carry one of the groups defined sub (a).

3. H-Tyr-D-Lys(For)-Gly-Phe-Met-O-$PEG_{3000}$-OMe or the hydrochloride thereof.

4. The peptide according to anyone of claims 1 to 3 for the use as a medicament.

5. The peptide according to anyone of claims 1 to 3 for the use as a remedy for increasing the motility of the muscles of the gastro-intestinal tract and of the urinary tract.

6. The peptide according to anyone of claims 1 to 3 for the use as a remedy for the treatment of paralytic symptoms of the gastro-intestinal tract and the urinary tract.

7. A process for preparing a peptide according to anyone of claims 1 to 6, characterized in that the amino acid sequences of the formula I are prepared by methods of peptide synthesis.

8. The use of a peptide of the formula I

$$A—Tyr—B—C—D—E \quad (I)$$

in which

A denotes hydrogen, alkyl, alkenyl, or alkenylcycloalkyl each having up to 7 carbon atoms, an α-amino acid or a peptide with 2 or 3 amino acids, preferably of the L-configuration,
B denotes an α-amino acid or α-amino-isobutyric acid,
C denotes glycine or azaglycine,
D denotes phenylalanine which can be substituted in the benzene nucleus by methyl, methoxy, halogen, nitro or trifluormethyl, dehydro- or hexahydrophenylalanine, aza- or N-methylphenylalanine, and
E denotes

a) $—O—R_1$ or 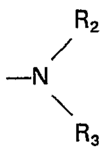

in which $R_1$, $R_2$ and $R_3$ are identical or different and are hydrogen, alkyl with 1 to 6 carbon atoms, cycloalkyl with 3 to 6 carbon atoms, alkenylcycloalkyl with 4 to 8 carbon atoms, hydroxyalkyl or dihydroxyalkyl, thioalkyl or mercaptoalkyl with 1 to 6 carbon atoms, aryl with 6 to 10 carbon atoms, aralkyl with 7 to 11 carbon atoms, or $R_2$ and $R_3$ together denote polymethylene with 2 to 6 carbon atoms, or $R_1$ denotes methyl-polyethylene glycol having a degree of polymerization of up to about 50,

b) an amino alcohol or an amino acid, which on its part may carry one of the groups defined sub a); or

c) homoserine or homocysteine thiolactone for the preparation of a pharmaceutical composition for stimulating the motility of the muscles of the gastro-intestinal tract and of the urinary tract.

9. The use of a peptide of the formula I, in which A, B, C and D are as defined in claim 2 and E is as defined in claim 1 sub (a) or (c) or is an α-amino alcohol or an α-amino acid each in the L- or D-configuration the side chain of which is optionally substituted like amino acid B and which on its part can carry on of the groups defined sub (a), for the preparation of a pharmaceutical composition for stimulating the motility of the muscles of the gastro-intestinal tract and of the urinary tract.

10. The use of a peptide of the formula I, in which A, B, C, D and E are as defined in claim 8 or 9, for the preparation of a pharmaceutical composition for the treatment of paralytic symptoms of the gastro-intestinal tract and the urinary tract.

**Claims for the Contracting State: AT**

1. A process for the preparation of a peptide of the formula I

$$A—Tyr—B—C—D—E \quad (I)$$

in which

A denotes hydrogen, alkyl, alkenyl, or alkenylcycloalkyl each having up to 7 carbon atoms, an α-amino acid or a peptide with 2 or 3 amino acids, preferably of the L-configuration,
B denotes an α-amino acid or α-amino-isobutyric acid,
C denotes glycine or azaglycine,
D denotes phenylalanine which can be substituted in the benzene nucleus by methyl, methoxy, halogen, nitro or trifluormethyl, dehydro- or hexahydrophenylalanine, aza- or N-methylphenylalanine, and
E denotes

a) $—O—R_1$ $R_1$ denotes a methyl-polyethylene glycol radical having a degree of polymerization of up to about 50,

b) an amino alcohol or an amino acid, which on its part may carry one of the groups defined sub (a), characterized in that the amino acid sequences of the formula I are prepared by methods of peptide synthesis.

2. A process according to claim 1 characterized in that a peptide of the formula I is prepared in which

A denotes hydrogen, alkyl, alkenyl or alkenylcycloalkyl each having up to 7 carbon atoms, an α-L-amino acid occurring in proteins or a simple metabolite thereof, or a di- or tripeptide consisting of these amino acids,
B denotes an α-D-amino acid which is an enantiomer of an amino acid occurring in nature, in the side chain of which an optionally existing amino group can be substituted by alkanoyl, cycloalkanoyl, aroyl, or aralkanoyl each having up to 10 carbon atoms, by alkyl-, cycloalkyl- or aralkyl-oxycarbonyl each having up to 7 carbon atoms,

optionally existing hydroxy groups or mercapto groups can be etherified by alkyl, aryl or aralkyl each having up to 10 carbon atoms, and carboxy groups can carry the groupings

$-O-R_1$ or 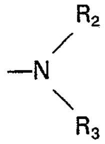

in which $R_1$, $R_2$ and $R_3$ are identical or different and are hydrogen, alkyl with 1 to 6 carbon atoms, cycloalkyl with 3 to 6 carbon atoms, alkenylcycloalkyl with 4 to 8 carbon atoms, hydroxyalkyl or dihydroxyalkyl, thioalkyl or mercaptoalkyl with 1 to 6 carbon atoms, aryl with 6 to 10 carbon atoms, aralkyl with 7 to 11 carbon atoms, and in addition $R_2$ and $R_3$ can together denote polymethylene with 2 to 6 carbon atoms, and, further, $R_1$ can denote methyl-polyethylene glycol having a degree of polymerization of up to about 50, in which optionally existing thioether functions can be converted to sulfoxide or sulfone and optionally existing aromatic radicals can be substituted by halogen, methyl, methoxy, nitro, or trifluoromethyl, norleucine or α-amino-isobutyric acid,
C denotes glycine or azaglycine,
D denotes phenylalanine which can be substituted in the benzene nucleus by methyl, methoxy, halogen, nitro or trifluormethyl, dehydro- or hexahydrophenylalanine, aza- or N-methylphenylalanine, and
E a) is as defined in claim 1 sub (a) or
b) is an α-amino alcohol or an α-amino acid each in the L- or D-configuration the side chain of which is optionally substituted like amino acid B and which on its part can carry one of the groups defined sub (a).

3. A process according to claim 1 or 2 characterized in that H-Tyr-D-Lys(For)-Gly-Phe-Met-O-$PEG_{3000}$-OMe is prepared.

4. A process according to anyone of claims 1 to 3 for the preparation of a peptide of the formula I for the use as a medicament.

5. A process according to anyone of claims 1 to 3 for the preparation of a peptide of the formula I for the use as a remedy for increasing the motility of the muscles of the gastro-intestinal tract and of the urinary tract.

6. A process according to anyone of claims 1 to 3 for the preparation of a peptide of the formula I for the use as a remedy for the treatment of paralytic symptoms of the gastro-intestinal tract and the urinary tract.

7. The use of a peptide of the formula I

$$A—Tyr—B—C—D—E \qquad (I)$$

in which

A denotes hydrogen, alkyl, alkenyl, or alkenylcycloalkyl each having up to 7 carbon atoms, an α-amino acid or a peptide with 2 or 3 amino acids, preferably of the L-configuration,
B denotes an α-amino acid or α-amino-isobutyric acid,
C denotes glycine or azaglycine,
D denotes phenylalanine which can be substituted in the benzene nucleus by methyl, methoxy, halogen, nitro or trifluormethyl, dehydro- or hexahydrophenylalanine, aza- or N-methylphenylalanine, and
E denotes

a) $-O-R_1$ or 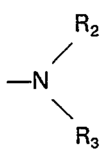

in which $R_1$, $R_2$ and $R_3$ are identical or different and are hydrogen, alkyl with 1 to 6 carbon atoms, cycloalkyl with 3 to 6 carbon atoms, alkenylcycloalkyl with 4 to 8 carbon atoms, hydroxyalkyl or dihydroxyalkyl, thioalkyl or mercaptoalkyl with 1 to 6 carbon atoms, aryl with 6 to 10 carbon atoms,

aralkyl with 7 to 11 carbon atoms, or $R_2$ and $R_3$ together denote polymethylene with 2 to 6 carbon atoms, or $R_1$ denotes methyl-polyethylene glycol having a degree of polymerization of up to about 50,
b) an amino alcohol or an amino acid, which on its part may carry one of the groups defined sub (a); or
c) homoserine or homocysteine thiolactone for the preparation of a pharmaceutical composition for stimulating the motility of the muscles of the gastro-intestinal tract and of the urinary tract.

8. The use of a peptide of the formula I, in which A, B, C and D are as defined in claim 2 and E is as defined in claim 1 sub (a) or (c) or is an α-amino alcohol or an α-amino acid each in the L- or D-configuration the side chain of which is optionally substituted like amino acid B and which on its part can carry one of the groups defined sub (a), for the preparation of a pharmaceutical composition for stimulating the motility of the muscles of the gastro-intestinal tract and of the urinary tract.

9. The use of a peptide of the formula I, in which A, B, C, D and E are as defined in claim 8 or 9, for the preparation of a pharmaceutical composition for the treatment of paralytic symptoms of the gastro-intestinal tract and the urinary tract.